# EUROPÄISCHE PATENTSCHRIFT

(11) **EP 3 231 462 B1**
(45) Veröffentlichungstag und Bekanntmachung des Hinweises auf die Patenterteilung: **18.05.2022**
(21) Anmeldenummer: 17165288.6
(22) Anmeldetag: 06.04.2017
(51) Int. Cl.: A61M 1/14

(54) **MEDIZINTECHNISCHES GERÄT MIT MONITOREINRICHTUNG**
MEDICAL DEVICE WITH MONITORING DEVICE
APPAREIL MÉDICAL COMPRENANT UN MONITEUR

(30) Priorität: 12.04.2016 DE 102016106655
(43) Veröffentlichungstag der Anmeldung: 18.10.2017
(73) Patentinhaber: B. Braun Avitum AG, 34212 Melsungen (DE)
(72) Erfinder: SCHÄFER, Oliver, 36286 Neuenstein (DE); SCHLEICHER, Christian, 36160 Dipperz (DE)
(74) Vertreter: Winter, Brandl - Partnerschaft mbB

(56) Entgegenhaltungen:
- WO-A1-2014/173772
- DE-A1-102013 005 743
- GB-A- 2 007 907
- US-A1- 2002 048 089
- US-A1- 2013 197 303
- US-A1- 2014 111 736
- US-B1- 8 830 174

## Beschreibung

Die Erfindung betrifft ein medizintechnisches Gerät mit einer Monitoreinrichtung und bezieht sich insbesondere auf eine Vorrichtung zur extrakorporalen Blutbehandlung, wie beispielsweise eine Dialysemaschine, die eine Monitoreinrichtung beispielsweise in Form eines Bildschirms mit berührungsempfindlicher Oberfläche beinhaltet.

DE 10 2013 005 743 A1 offenbart ein Dialysegerät mit einem transparenten Display, durch das Teile des Geräts sichtbar sind und auf dem Informationen angezeigt werden können.

US 2002/0048089 A1 beschreibt ein medizinisches Gerät mit einer Anzeigevorrichtung, die ein flaches oder gekrümmtes Display aufweist und auf der ein Patient während einer Behandlung Videos anschauen kann.

US 2013/0197303 A1 offenbart eine medizinische Gesichtsmaske mit einem Intubationsanschluss und einem Display, das auf der Maskenoberfläche angeordnet ist und entsprechend der Maskenoberfläche eine Krümmung zeigt.

WO 2014/173772 A1 beschreibt eine Sensoreinrichtung für einen Insulin-Pen, in die ein gekrümmtes OLED Display eingebaut ist.

US 8,830,174 B1 offenbart ein elektronisches Gerät, beispielsweise Handys, eBooks, Tablets, mit einem Display, das gekrümmt ausgebildet sein kann.

Speziell bei feststehenden, d.h. nicht drehbaren oder neigbaren Monitorbaugruppen, sind diese Lösungen dahingehend nachteilig, dass durch eine Blendwirkung eine erhebliche Beeinträchtigung der Sichtbarkeit und/oder Ablesbarkeit des dargestellten Monitorinhaltes auftreten kann.

Der Erfindung liegt daher als eine Aufgabe zugrunde, ein medizintechnisches Gerät mit einer Monitoreinrichtung zu schaffen, welche Spiegelungen reduziert und/oder die Blendfreiheit bei einer feststehenden Monitoreinrichtung erhöht und eine verbesserte Sichtbarkeit und/oder Ablesbarkeit von auf der Monitoreinrichtung dargestellten Inhalten bereitstellt.

Speziell soll für eine Vorrichtung zur extrakorporalen Blutbehandlung, insbesondere eine Dialysemaschine, eine solche Monitoreinrichtung bereitgestellt werden.

Diese Aufgabe wird erfindungsgemäß gelöst durch ein medizintechnisches Gerät mit den Merkmalen des Anspruchs 1. Vorteilhafte Weiterbildungen der Erfindung sind Gegenstand der beigefügten Unteransprüche.

Der Erfindung liegt als eine allgemeine Idee zugrunde, an einem Gerät für die Medizintechnik und insbesondere einem Gerät zur extrakorporalen Blutbehandlung, wie beispielsweise einer Dialysemaschine und dergleichen, eine gebogen, gewölbt oder gekrümmt, beispielsweise konkav in Bezug auf einen Betrachter/Bediener/Anwender, ausgeformte Monitoreinrichtung in Form beispielsweise eines Bildschirms mit entsprechender Formgebung einer Anzeige- und/oder Bedienoberfläche einschließlich gegebenenfalls grafischer Benutzeroberfläche anzuordnen. Eine auf die Monitoroberfläche gelegte berührungsempfindliche Folie (Touchfolie) kann in Verbindung mit dem darunter liegenden Anzeigefeld, beispielsweise einer TFT-Einrichtung, sowohl eine grafische Darstellung therapierelevanter Parameter als auch die Einstellung verschiedener Parameter erlauben. Die konkav (als konkaves Display) ausgebildete Monitoreinrichtung reduziert deutlich Blendwirkungen und Blendeffekte. Eine konkave Monitoroberfläche mit integrierter Touchfolie ist weiter dahingehend vorteilhaft, dass nicht nur Blendeffekte reduziert werden und somit aufgrund der reduzierten Blendeffekte eine angenehmere Informationsdarstellung möglich wird, sondern auch die Gefahr möglicher Oberflächenbeschädigungen des Displays verringert wird.

Spezielle Vorteile resultieren aus einer Anordnung an Geräten bzw. Maschinen mit feststehendem Monitor, da bei diesen ein Verdrehen des Monitors gegenüber einer störenden Lichtquelle nur bedingt möglich ist, da für ein Verdrehen des Monitors die gesamte Maschine gedreht werden muss. Insoweit kann bei einem konkav ausgebildeten Monitor eine Drehfunktion des Monitors entfallen, da ungünstiger Lichteinfall auf den Monitor gestreut und daher den Anwender nicht stören wird.

Im Einzelnen werden die vorstehenden Vorteile realisiert und die Aufgabe gelöst durch ein medizintechnisches Gerät mit einem Korpus und einer Monitoreinrichtung als einer Anzeige- und Bedieneinrichtung, die eine konkav gekrümmte Anzeige- und Bedienoberfläche aufweist.

Die Konkavität der Oberfläche ist auf den normalen Bedienerabstand des Anwenders zur Oberfläche des Monitors abgestimmt. In diesem Fall wird durch die gekrümmte Form der Monitoroberfläche das Licht nicht zum Auge des Anwenders reflektiert, sondern in alle Richtungen gestreut. Gleichzeitig wirft bei Beleuchtung aus rückwärtiger Sicht des Anwenders dessen Körper einen Schatten auf den konkaven Monitor, der kontrasterhöhend wirkt. Der Kontrast kann durch Verwendung einer OLED-Technologie in Verbindung mit einem gekrümmten Display nochmals weiter erhöht werden.

Außerdem ist der Grad und/oder der Verlauf der Krümmung der Anzeige- und Bedienoberfläche dazu vorgesehen und angepasst, Reflektionen von Lichtstrahlen in einem vorbestimmten Bedienerabstand zu der Anzeige- und Bedienoberfläche zum Auge des Bedieners hin zu reduzieren.

Weiter ist die Konkavität der Krümmung der Anzeige- und Bedieneinrichtung an eine vorbestimmte Entfernung von einem Anwender zu dem Gerät angepasst, die innerhalb eines Bedienbereichs bzw. innerhalb einer durchschnittlichen Armlänge eines Benutzers in einem Bereich zwischen 0,2m und 1m liegt, besonders vorzugsweise in einem Bereich zwischen 0,3m und 0,7m.

Vorzugsweise ist die Anzeige- und Bedienoberfläche gleichmäßig oder stetig gekrümmt.

Bevorzugt ist das medizintechnische Gerät als eine Vorrichtung zur extrakorporalen Blutbehandlung ausgebildet mit einem Gehäuse als den Korpus zur Aufnahme betrieblicher Komponenten für die extrakorporale Blutbehandlung. Weiter bevorzugt ist das medizintechnische Gerät als eine Dialysemaschine ausgebildet.

Bevorzugt ist die Anzeige- und Bedienoberfläche um zumindest eine Raumachse konkav gekrümmt ausgebildet. Hierbei kann die Raumachse zumindest eine horizontal oder vertikal verlaufende Raumachse sein.

Auch bevorzugt kann die Anzeige- und Bedienoberfläche der Monitoreinrichtung eine Vielzahl von konkav oder konvex gekrümmten Teilabschnitten aufweisen.

Bevorzugt kann die Anzeige- und Bedieneinrichtung Teil einer an einer Oberfläche des Gehäuses festgelegten und gegenüber dieser vorstehenden Monitoreinrichtung sein.

Auch bevorzugt kann die Anzeige- und Bedieneinrichtung bzw. die Anzeige- und Bedienoberfläche in das Gehäuse integriert aufgenommen sein.

Bevorzugt kann eine Mehrzahl von Monitoreinrichtungen mit jeweils zumindest einer Anzeige- und Bedienoberfläche vorgesehen und zumindest eine Monitoreinrichtung eine extern an das Gehäuse angelenkte oder eine integriert in das Gehäuse aufgenommene Monitoreinrichtung sein.

Bevorzugt ist die Anzeige- und Bedieneinrichtung eine nach dem OLED- oder AMOLED-Prinzip arbeitende Anzeige- und Bedieneinrichtung.

Weiter bevorzugt ist der Grad und der Verlauf der Krümmung der Anzeige- und Bedienoberfläche dazu vorgesehen und angepasst, Lichtstrahlen zu streuen, insbesondere durch Einsatz einer streuwirkungsunterstützenden strukturierten und der Anzeige- und Bedienoberfläche vorgelagerten Schicht.

Die Erfindung wird nachstehend anhand bevorzugter Ausführungsbeispiele unter Bezugnahme auf die beigefügte Zeichnung näher beschrieben. Es zeigen:

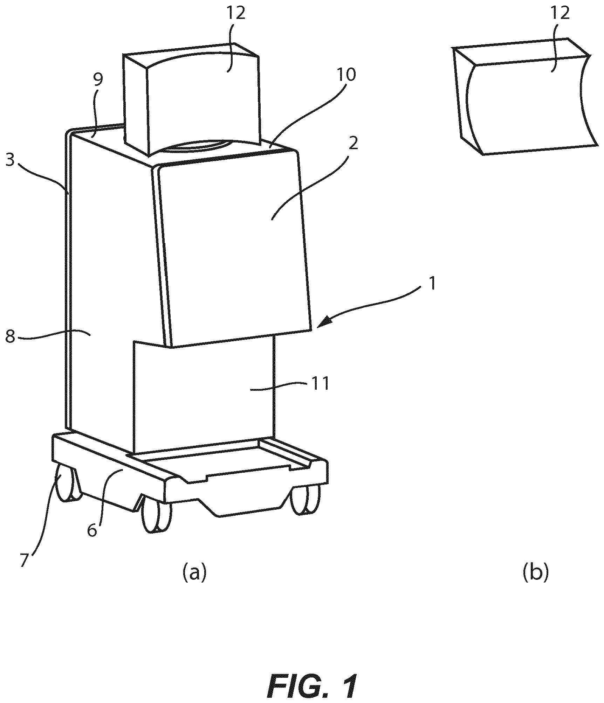

Fig. 1a in vereinfachter, perspektivischer Darstellung ein Gerätegehäuse einer Dialysemaschine als einem medizintechnischen Gerät zur extrakorporalen Blutbehandlung mit einer extern eines Korpus angeordneten Anzeige- und/oder Bedieneinrichtung, und Fig. 1b die Anzeige- und/oder Bedieneinrichtung in modifizierter Anordnung bzw. Ausrichtung gemäß einem ersten Ausführungsbeispiel; und

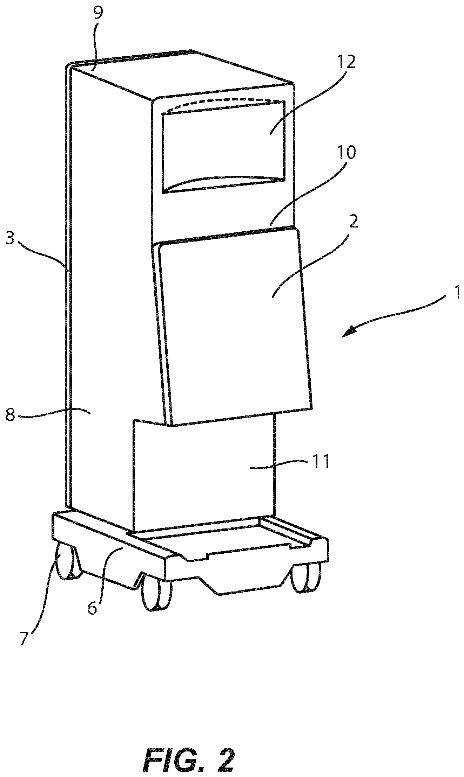

Fig. 2 zeigt in vereinfachter, perspektivischer Darstellung ein Gerätegehäuse einer Dialysemaschine als einem medizintechnischen Gerät zur extrakorporalen Blutbehandlung mit einer intern in einem Korpus angeordneten Anzeige- und/oder Bedieneinrichtung gemäß einem zweiten Ausführungsbeispiel.

In der nachfolgenden Figurenbeschreibung werden in den einzelnen Figuren gleiche oder gleichwirkende Elemente und/oder Komponenten gleich und/oder mit denselben Bezugszeichen bezeichnet und zweckmäßig nicht redundant beschrieben. In Fällen, in welchen ein nachfolgendes Ausführungsbeispiel funktionell zumindest einem vorangehenden entspricht, d.h. entsprechende Funktionen, Anordnungen und/oder Verfahrens- oder Betriebsabläufe gleichermaßen umfasst sind, wird zweckmäßig nur auf Unterschiede eingegangen.

Fig. 1a zeigt in vereinfachter, perspektivischer Darstellung ein Gerätegehäuse einer Dialysemaschine.

Gemäß Fig. 1a hat die Dialysemaschine ein Geräte- bzw. Maschinengehäuse 1 (nachstehend als Korpus oder Gehäuse bezeichnet), in welchem eine Anzahl von Komponenten zur Durchführung einer Blutbehandlung in an sich bekannter Weise funktionsfähig zusammenwirkend angeordnet sind oder angeordnet werden können, wie beispielsweise eine Steuerelektronik, zumindest Teile der Hydraulik, Pumpen, Heizeinrichtungen und ggf. Tanks oder Beutel für ausgewählte Lösungen, welche sämtlich aus dem Stand der Technik hinlänglich bekannt sind und daher nicht weiter dargestellt oder beschrieben werden.

Das Gehäuse 1 kann beispielsweise im Wesentlichen zumindest zwei Seitenplatten 8 , eine zumindest teilweise als schwenkbare Tür/Klappe ausgebildete Rückwand 3 , eine Haube 9, welche die beiden vorzugsweise parallel beabstandeten Seitenplatten 8 oberseitig verbindet, eine Bodenplatte (beispielsweise als Teil eines Gerätesockels) 6, welche die beiden Seitenplatten 8 bodenseitig verbindet, und eine zumindest teilweise als schwenkbare Tür/Klappe 2 ausgebildete Vorderwand aufweisen.

An der Bodenplatte 6 können Geräterollen 7 montierbar sein, welche zusammen mit der Bodenplatte 6 ein geräteinternes (integriertes) Fahrwerk einer mobilen Dialysemaschine bilden können. Außerdem können die Haube 9 und der Gerätesockel bzw. die Bodenplatte 6 an den Ober- und Unterkanten der beiden Seitenplatten 8 festgelegt, beispielsweise über zumindest Teile der Kantenlänge hinweg versteifend angeschweißt, sein. Auf diese Weise kann durch die beiden Seitenplatten 8, die Haube 9, die Bodenplatte 6 und eine Frontplatte 11 ein verwindungssteifes und über zu öffnende Teile innenseitig zugängliches Gehäuse 1 bereitgestellt sein.

In diesem Ausführungsbeispiel ist an der Haube 9 zentral oder mittig ein Anschlusssockel 10 angeformt, an welchem eine Monitoreinrichtung in Form eines Monitors montiert ist. Die Monitoreinrichtung kann mit einer Anzeige- und/oder Bedienoberfläche 12 zur lediglichen Darstellung von Information ausgelegt sein, oder alternativ (nicht dargestellt) eine Anzeige- und/oder Bedienoberfläche sein, die zumindest einen Informationsanzeigeabschnitt und zumindest einen Bedienabschnitt (grafische Benutzeroberfläche) mit einer Reihe von Bedienelementen zur Bedienung der Maschine beinhaltet. Vorzugsweise ist in diesem Fall zumindest ein Teil der Monitoreinrichtung berührungsempfindlich ausgebildet, beispielsweise mittels einer über einer Dünnfilmtransistoranordnung liegenden, resistiv oder kapazitiv berührungsempfindlichen Folie oder Schicht. Berührungsempfindliche Anzeige- und/oder Bedieneinrichtungen sind an sich bekannt und werden daher nicht näher beschrieben.

In diesem Ausführungsbeispiel ist weiter die gesamte Monitoreinrichtung, zumindest aber ein Teil ihrer Anzeige- und/oder Bedienoberfläche 12 um eine Raumachse, vorzugsweise um eine Höhenrichtung bzw. um eine vertikal verlaufende Achse, konkav, d.h. von einem vor dem Gerät stehenden Anwender weg, wie in Fig. 1a gezeigt, gebogen oder gekrümmt ausgebildet.

Fig. 1b zeigt die Anzeige- und/oder Bedieneinrichtung in modifizierter Anordnung bzw. Ausrichtung gemäß dem ersten Ausführungsbeispiel. Gemäß Fig. 1b kann die Monitoreinrichtung in gegenüber der Fig. 1a modifizierter Anordnung bzw. Ausrichtung um eine andere Raumachse, beispielsweise in Seitenrichtung bzw. um eine horizontal verlaufende Achse, konkav ausgebildet sein.

In einer anderen, nicht dargestellten Modifikation kann die Monitoreinrichtung auch mehrere, jeweils separat gekrümmte Teilabschnitte aufweisen, beispielsweise mehrere konkave Abschnitte und/oder konvexe Abschnitte in vorbestimmter Anordnung oder Verteilung, beispielsweise in einer Wellenform, über die Anzeige- und/oder Bedienoberfläche 12 der Monitoreinrichtung. In einer weiteren, nicht dargestellten Modifikation kann darüber hinaus die Monitoreinrichtung ausgehend von jeweils vorbestimmten Punkten, beispielsweise den Eckpunkten einer rechteckförmigen Anzeige- und/oder Bedienoberfläche 12, nach Art einer an diesen Punkten zum Anwender hin gezogenen und im übrigen Bereich tiefer liegenden Wanne sowohl in Höhenrichtung als auch in Seitenrichtung konkav gekrümmt sein.

Fig. 2 zeigt in vereinfachter, perspektivischer Darstellung ein Gerätegehäuse einer Dialysemaschine als einem medizintechnischen Gerät zur extrakorporalen Blutbehandlung mit einer intern in einem Korpus angeordneten Anzeige- und/oder Bedieneinrichtung gemäß einem zweiten Ausführungsbeispiel.

In dem zweiten Ausführungsbeispiel gemäß Fig. 2 ist der Monitor mit der Anzeige- und/oder Bedieneinrichtung 12 nicht auf der Oberseite des Korpus des Gehäuses 1 aufmontiert, sondern in dem Korpus des Gehäuses 1 integriert aufgenommen und beispielsweise an vorbestimmter Stelle mittels einer Rahmenvorrichtung oder Halteeinrichtung (Halterung) rückseitig an der Frontplatte 11 festgelegt. In dem zweiten Ausführungsbeispiel sind damit dieselben Konfigurationen, Ausführungsformen und Modifikationen wie in dem ersten Ausführungsbeispiel möglich.

Bevorzugt kann darüber hinaus in dem ersten und dem zweiten Ausführungsbeispiel der Grad und die Form bzw. der Verlauf der Konkavität der Anzeige- und/oder Bedienoberfläche 12 auf einen vorbestimmten Abstand des Anwenders zu beispielsweise einer Oberfläche des die Anzeige- und/oder Bedienoberfläche 12 aufnehmenden oder beinhaltenden Monitors kontrasterhöhend derart angepasst sein, dass durch die gekrümmte Form der Oberfläche Licht in geringerem Ausmaß zum Auge des Anwenders reflektiert, sondern vielmehr diversifizierend gestreut wird. Zur Steigerung der Streuwirkung kann der Oberfläche der Monitoreinrichtung eine geeignet strukturierte, vorzugsweise transparente oder geeignet opake, Deckschicht, Filterfolie oder dergleichen vorgelagert sein.

Die Anordnung einer Lichtquelle hinter dem Anwender kann durch Lichteinfall an dem Anwender vorbei und Schattenwurf durch dessen Körper weiter kontrasterhöhend sein. Eine zusätzliche Kontrasterhöhung kann ferner durch vorzugsweise Verwendung der an sich bekannten OLED- oder AMOLED-Technologie in Verbindung mit der gekrümmten Oberfläche des Monitors bzw. der Monitoreinrichtung erzielt werden.

Vorteile resultieren gemäß dem ersten und dem zweiten Ausführungsbeispiel insbesondere bei einer Anordnung der gewölbten Monitoreinrichtung an Geräten bzw. Maschinen mit feststehend oder integriert angeordnetem Monitor, da bei solchen Maschinen ein Drehen des Monitors gegenüber einer störenden Lichtquelle nur bedingt möglich ist, weil in diesem Fall die gesamte Maschine gedreht werden müsste. Insoweit kann durch die Anordnung eines konkav ausgebildeten Monitors eine Drehfunktion des Monitors weggelassen werden, weil ungünstig oder störend reflektierender Lichteinfall auf den Monitor Spiegelungen und/oder Blendwirkungen verringernd und den Anwender nicht weiter störend gestreut wird.

Wie vorstehend beschrieben wurde, kann gemäß dem ersten Ausführungsbeispiel eine vorbestimmte und/oder angepasste Monitor-System-Baugruppe vorgesehen sein, die auf ein (auch bereits bestehendes und damit nachrüstbares) Gehäuse 1 aufsetzbar ist. Gemäß dem zweiten Ausführungsbeispiel ist ein feststehender Monitor direkt in ein Gehäuse 1 integrierbar. Darüber hinaus sind zumindest Teile des ersten und des zweiten Ausführungsbeispiels kombinierbar. Beispielsweise ist ein (zusätzlicher und/oder externer) Neben- oder Submonitor, welcher beispielsweise an anderer Stelle integriert sein oder mittels einer gegebenenfalls verschwenkbaren Armvorrichtung an dem Gehäuse 1 angebracht sein kann, darstellbar.

Es wird angemerkt, dass vergleichbare Wirkungen und Vorteile auch mit einer konvex, d.h. zum Anwender hin, gekrümmten Oberfläche der Monitoreinrichtung erzielbar sind.

Vorstehend wurde somit ein medizintechnisches Gerät beschrieben, das einen Korpus und eine Anzeige- und/oder Bedieneinrichtung, die eine gekrümmte Anzeige- und/oder Bedienoberfläche 12 aufweist, beinhaltet. Das medizintechnische Gerät ist vorzugsweise als eine Vorrichtung zur extrakorporalen Blutbehandlung und stärker vorzugsweise als eine Dialysemaschine ausgebildet, mit einem Gehäuse 1 als den Korpus zur Aufnahme betrieblicher Komponenten für die extrakorporale Blutbehandlung. Die Anzeige- und/oder Bedienoberfläche (12) ist um zumindest eine Raumachse, bevorzugt eine vertikal oder horizontal verlaufende Raumachse konkav oder konvex gekrümmt ausgebildet.

Es versteht sich, dass die Erfindung nicht auf die beschriebenen Ausführungsbeispiele beschränkt ist, sondern dass sich innerhalb des durch die nachstehenden Ansprüche definierten Schutzumfangs für den Fachmann gleichwohl naheliegend Kombinationen von zumindest Teilen dieser Ausführungsbeispiele, Modifikationen und Äquivalente ergeben können.

## Patentansprüche

1. Medizintechnisches Gerät mit einem Korpus und einer Monitoreinrichtung, wobei die Monitoreinrichtung eine Anzeige- und Bedieneinrichtung ist, die eine gekrümmte Anzeige- und Bedienoberfläche (12) aufweist, und **gekennzeichnet dadurch, dass** die gekrümmte Anzeige- und Bedienoberfläche (12) eine konkave Krümmung aufweist und der Grad und/oder der Verlauf der konkaven Krümmung der Anzeige- und Bedienoberfläche (12) auf einen vorbestimmten Bedienerabstand des Anwenders zur Anzeige- und Bedienoberfläche (12) der Monitoreinrichtung abgestimmt ist, um Reflektionen von Lichtstrahlen in dem vorbestimmten Bedienerabstand zu der Anzeige- und Bedienoberfläche (12) zum Auge des Bedieners hin zu reduzieren, wobei der vorbestimmte Bedienerabstand in einem Bereich zwischen 0,2 m und 1 m liegt.

2. Medizintechnisches Gerät nach Anspruch 1, das als eine Vorrichtung zur extrakorporalen Blutbehandlung, insbesondere als eine Dialysemaschine, ausgebildet ist, mit einem Gehäuse (1) als den Korpus zur Aufnahme betrieblicher Komponenten für die extrakorporale Blutbehandlung.

3. Medizintechnisches Gerät nach einem der vorangehenden Ansprüche, bei dem die Anzeige- und Bedienoberfläche (12) um zumindest eine, insbesondere horizontal oder vertikal verlaufende, Raumachse konkav gekrümmt ausgebildet ist.

4. Medizintechnisches Gerät nach einem der vorangehenden Ansprüche, bei dem die Anzeige- und Bedienoberfläche (12) der Monitoreinrichtung eine Vielzahl von separat konkav oder konvex gekrümmten Teilabschnitten aufweist.

5. Medizintechnisches Gerät nach Anspruch 4, wobei die Vielzahl von separat konkav oder konvex gekrümmten Teilabschnitten in einer Wellenform angeordnet ist.

6. Medizintechnisches Gerät nach einem der vorangehenden Ansprüche, bei dem die Anzeige- und Bedienoberfläche (12) Teil einer an einer Oberfläche des Gehäuses (1) festgelegten und gegenüber dieser vorstehenden Monitoreinrichtung ist.

7. Medizintechnisches Gerät nach einem der vorangehenden Ansprüche, bei dem die Anzeige- und Bedienoberfläche (12) in das Gehäuse (1) integriert aufgenommen ist.

8. Medizintechnisches Gerät nach einem der Ansprüche 1 bis 6, bei dem eine Mehrzahl von Monitoreinrichtungen mit jeweils zumindest einer Anzeige- und Bedienoberfläche (12) vorgesehen sind und zumindest eine Monitoreinrichtung eine extern an das Gehäuse (1) angelenkte oder integriert in das Gehäuse (1) aufgenommene Monitoreinrichtung ist.

9. Medizintechnisches Gerät nach einem der vorangehenden Ansprüche, bei dem die Anzeige- und Bedieneinrichtung eine nach dem OLED- oder AMOLED-Prinzip arbeitende Anzeige- und Bedieneinrichtung ist.

10. Medizintechnisches Gerät nach einem der vorangehenden Ansprüche, wobei der Anzeige- und Bedienoberfläche (12) eine streuwirkungsunterstützende, strukturierte Schicht vorgelagert ist.

11. Medizintechnisches Gerät nach einem der vorangehenden Ansprüche, wobei die Monitoreinrichtung ausgehend von jeweils vorbestimmten Punkten nach Art einer an diesen Punkten zum Anwender hin gezogenen und übrigen Bereich tiefer liegenden Wanne in Höhenrichtung und in Seitenrichtung konkav gekrümmt ist.

## Claims

1. A medical device comprising a body and a monitor system, wherein the monitor system is a display and operating unit which includes a curved display and user interface (12), **characterized in that** the curved display and user interface (12) has a concave curvature and the degree and/or the course of the concave curvature of the display and user interface (12) is adapted to a predetermined user distance of the user from the display and user interface (12) of the monitor system in order to reduce reflections of light rays at the predetermined user distance from the display and user interface (12) towards the eye of the user, wherein the predetermined user distance is within a range of 0.2 m and 1 m.

2. The medical device according to claim 1 in the form of an apparatus for extracorporeal blood treatment, in particular a dialysis machine, comprising a housing (1) constituting the body for accommodating operational components for the extracorporeal blood treatment.

3. The medical device according to one of the preceding claims, wherein the display and user interface (12) is designed to be concavely curved about at least one, especially horizontally or vertically extending, space axis.

4. The medical device according to one of the preceding claims, wherein the display and user interface (12) of the monitor system includes a plurality of separately concavely or convexly curved segments.

5. The medical device according to claim 4, wherein the plurality of separately concavely or convexly curved segments is arranged in a waveform.

6. The medical device according to one of the preceding claims, wherein the display and user interface (12) is part of a monitor system being fixed to a surface of the housing (1) and projecting from the latter.

7. The medical device according to one of the preceding claims, wherein the display and user interface (12) is integrated in the housing (1).

8. The medical device according to one of claims 1 to 6, wherein a plurality of monitor systems each including at least one display and user interface (12) is provided and at least one monitor system is a monitor system which is externally linked to the housing (1) or is integrated in the housing (1).

9. The medical device according to one of the preceding claims, wherein the display and operating unit is a display and operating unit which functions on the basis of the OLED or AMOLED principle.

10. The medical device according to one of the preceding claims, wherein the display and user interface (12) is preceded by a scattering effect-supporting, structured layer.

11. The medical device according to one of the preceding claims, wherein the monitor device is concavely curved in the height direction and in the lateral direction starting from respective predetermined points in the manner of a trough drawn towards the user at these points and lying lower in the remaining region.

## Revendications

1. Appareil médico-technique avec un corps et équipement de pilotage, dans lequel l'équipement de pilotage est un équipement d'affichage et de commande qui présente une surface d'affichage et de commande (12) courbée, et **caractérisé en ce que** la surface d'affichage et de commande (12) courbée présente une courbure concave et le degré ou le tracé de la courbure concave de la surface d'affichage et de commande (12) concorde avec une distance d'opérateur prédéterminée de l'utilisateur par rapport à la surface d'affichage et de commande (12) de l'équipement de pilotage pour réduire des reflets de rayons lumineux dans la distance d'opérateur prédéterminée par rapport à la surface d'affichage et de commande (12) en allant vers l'œil de l'utilisateur, dans lequel la distance d'opérateur prédéterminée se situe dans une plage entre 0,2 m et 1 m.

2. Appareil médico-technique selon la revendication 1, qui est conçu en tant que dispositif pour le traitement extracorporel du sang, en particulier en tant que machine de dialyse, avec un logement (1) en tant que corps pour recevoir des composantes opérationnelles pour le traitement extracorporel du sang.

3. Appareil médico-technique selon l'une des revendications précédentes, dans lequel la surface d'affichage et de commande (12) est conçue avec une courbure concave autour d'au moins un axe spatial qui se déroule en particulier à l'horizontale ou à la verticale.

4. Appareil médico-technique selon l'une des revendications précédentes, dans lequel la surface d'affichage et de commande (12) de l'équipement de pilotage présente une pluralité de sections partielles courbées séparément concaves ou convexes.

5. Appareil médico-technique selon la revendication 4, dans lequel la pluralité de sections partielles courbées séparément concaves ou convexes sont agencées dans une forme d'onde.

6. Appareil médico-technique selon l'une des revendications précédentes, dans lequel la surface d'affichage et de commande (12) fait partie d'un équipement de pilotage fixé contre une surface du logement (1) et qui précède celle-ci.

7. Appareil médico-technique selon l'une des revendications précédentes, dans lequel la surface d'affichage et de commande (12) est reçue dans le logement (1) de façon intégrée.

8. Appareil médico-technique selon l'une des revendications 1 à 6, dans lequel sont prévus une pluralité d'équipements de pilotage avec respectivement au moins une surface d'affichage et de commande (12), et au moins un équipement de pilotage est un équipement de pilotage reçu de façon articulée extérieurement contre le logement (1) ou de façon intégrée dans le logement (1).

9. Appareil médico-technique selon l'une des revendications précédentes, dans lequel l'équipement d'affichage et de commande est un équipement d'affichage et de commande qui travaille selon le principe OLED ou AMOLED.

10. Appareil médico-technique selon l'une des revendications précédentes, dans lequel une couche structurelle qui soutient un effet de diffusion est montée en amont de la surface d'affichage et de commande (12).

11. Appareil médico-technique selon l'une des revendications précédentes, dans lequel l'équipement de pilotage est courbé de façon concave dans le sens de la hauteur et dans la direction latérale en partant de points prédéterminés respectifs, à la manière d'un bac tiré au niveau de ces points en allant vers l'utilisateur et avec une région restante qui se situe plus en profondeur.
